Europäisches Patentamt

European Patent Office

Office européen des brevets

(19)

(11) **EP 0 604 884 B1**

(12) **EUROPÄISCHE PATENTSCHRIFT**

(45) Veröffentlichungstag und Bekanntmachung des Hinweises auf die Patenterteilung:
**07.05.1997 Patentblatt 1997/19**

(51) Int Cl.6: **C07D 323/06**

(21) Anmeldenummer: **93120599.1**

(22) Anmeldetag: **21.12.1993**

(54) **Verfahren zur Herstellung von Trioxan**

Process for the preparation of trioxane

Procédé de préparation de trioxanne

(84) Benannte Vertragsstaaten:
**DE ES FR GB IT NL**

(30) Priorität: **31.12.1992 DE 4244582**
**05.11.1993 DE 4337706**

(43) Veröffentlichungstag der Anmeldung:
**06.07.1994 Patentblatt 1994/27**

(73) Patentinhaber: **HOECHST AKTIENGESELLSCHAFT**
**65929 Frankfurt am Main (DE)**

(72) Erfinder:
• **Emig, Gerhard, Prof.Dr.**
**D-91058 Erlangen (DE)**

• **Krüger, Benno, Dr.**
**D-65529 Waldems-Esch (DE)**
• **Kern, Frank, Dr.**
**D-76870 Kandel (DE)**
• **Hoffmockel, Michael, Dr.**
**D-65527 Niedernhausen (DE)**
• **Mück, Karl-Friedrich, Dr.**
**D-65207 Wiesbaden (DE)**
• **Sextro, Günter, Dr.**
**D-65207 Wiesbaden (DE)**

(56) Entgegenhaltungen:
DE-A- 3 106 476        FR-A- 1 526 132
GB-A- 1 045 639

**Beschreibung**

Die Erfindung bezieht sich auf ein Verfahren zur kontinuierlichen Herstellung von Trioxan in der Gasphase durch heterogene Katalyse mit Vanadylhydrogenphosphat-Hemihydrat (VO) $HPO_4$ * 1/2 $H_2O$ als Katalysator. Bei einer Abwandlung des Verfahrens wird der Katalysator vor seinem Einsatz durch Wasserdampfbehandlung aktiviert.

Trioxan kann aus wäßrigen Formaldehydlösungen mit sauren Katalysatoren hergestellt werden. Beispielsweise wird in DE-A-31 06 476 die Herstellung von Trioxan aus wäßrigen Formaldehydlösun en durch Erhitzen in Gegenwart von Heteropolysäuren beschrieben. Kennzeichnend für diese Prozesse ist der hohe Energieverbrauch zum Verdampfen von Wasser, das durch die Eduktströme in den Prozeß eingetragen wird. Es gibt verschiedene Vorschläge, Trioxan durch eine Gasphasentrimerisierung von Formaldehyd herzustellen, jeboch wird hierbei immer mit Formaldehydströmen von unterschiedlichem Wassergehalt gearbeitet. Durch das Arbeiten mit wasserhaltigem Formaldehyd treten durch Ablagerungen von Polyoxymethylen auf der Katalysatorenoberfläche Probleme auf.

Ein Verfahren zur Herstellung von Trioxan mittels eines sauren Harzionentauschers ist bekannt (DE-C-1 593 990). Ebenfalls bekannt ist ein Katalysator für die Gasphasentrimerisierung in Form von Phosphorsäure und Schwefelsäure auf $SiO_2$-Trägern (GB-A-1045639).

Es bestand die Aufgabe, die genannten Nachteile zu beseitigen.

Gelöst wurde die Aufgabe durch ein Verfahren, das sich von den bisherigen Verfahren durch den Katalysator, die Zusammensetzung des Eduktstroms und die hohe Selektivität des Katalysators, vor allem bei hohen Formaldehydpartialdrücken unterscheidet.

Die Erfindung beschreibt somit ein Verfahren zur Herstellung von Trioxan aus Formaldehyd in der Gasphase in Gegenwart von Vanadylhydrogenphosphat-Hemihydrat als Katalysator. In einer besonderen Ausführungsform wird der Katalysator vor seinem Einsatz durch Behandlung mit Wasserdampf aktiviert.

Besonders hervorzuheben sind hierbei folgende Vorteile

1. Wesentliche Erhöhung der Raumzeitausbeuten (RZA) [kg/m$^3$ * h], insbesondere, wenn der Katalysator vorher mit Wasserdampf aktiviert wird.
2. Keine Nebenprodukte
3. Moderate Wärmeentwicklung im Synthesereaktor

Der Katalysator wird im allgemeinen in Substanz eingesetzt, d.h. ohne Träger oder Presshilfen. Seine Summenformel ist (VO) $HPO_4$ * 1/2 $H_2O$. Durch einen Verlust von Wasser unter Reaktionsbedingungen und die Bildung verschiedener meta-Phosphate bis hin zum Vanadylpyrophosphat, $(VO)_2P_2O_7$, d.h. wasserärmeren Formen der Verbindung, verändert sich die Aktivität und Selektivität nicht. Eine wesentliche Steigerung der Aktivität wird durch eine vorherige Behandlung des Katalysators mit Wasserdampf bei 100 bis 220°C erreicht. Wird im Verlaufe des Einsatzes bei einem kontinuierlichen Verfahren eine Aktivitätsminderung des Katalysators festgestellt, läßt sich diese durch eine Wasserdampfbehandlung rückgängig machen.

Durch den Einsatz des reinen Katalysators können Ablagerungen, wie diese bei der Verwendung von Phosphorsäure auf $SiO_2$ üblich sind, verhindert werden.

Im allgemeinen wird die Aktivierung bei Temperaturen von 100 bis 220°C, vorzugsweise 145 bis 200°C, insbesondere 165 bis 195°C über einen Zeitraum 24 Stunden bis 14 Tage durchgeführt. Niedrigere Temperaturen und geringe Behandlungszeiten bewirken keine Aktivierung des Katalysators. Höhere Temperaturen führen zur Zerstörung des Katalysators. Die Dauer der Behandlung kann praktisch unbegrenzt erfolgen, jedoch ist im allgemeinen eine wirtschaftlich vertretbare Zeit vorzusehen. Eine Abhängigkeit ist hier auch von der Dimension der Katalysatormenge vorhanden. Bei kleinen Katalysatormengen, z. B. auch im Versuchsmaßstab, kann die Zeit von 24 Stunden unterschritten werden. Sie ist auf den Einzelfall abzustimmen.

Zur Aktivierung dient Wasserdampf, der gegebenenfalls mit einem Trägergas versetzt ist. Im allgemeinen wird drucklos gearbeitet, es kann jedoch auch erhöhter Druck angewandt werden.

Der für das Verfahren einsetzbare Formaldehyd kann von unterschiedlichem Wassergehalt sein, d. h. bis zu 5 Gew.-% Wasser enthalten. Bevorzugt ist wasserfreier Formaldehyd.

Der Temperaturbereich für die Reaktion liegt bei 80 bis 150°C. Bevorzugt ist der Bereich von 100 bis 120°C.

Die Reaktion wird durch den Partialdruck des Formaldehydes beeinflußt. Der Katalysator hat über einen großen Druckbereich eine hohe Selektivität für die Bildung von Trioxan. Der Eingangspartialdruck des Formaldehydes beträgt im allgemeinen 0,5 bis 5 bar, vorzugsweise 0,5 bis 2 bar.

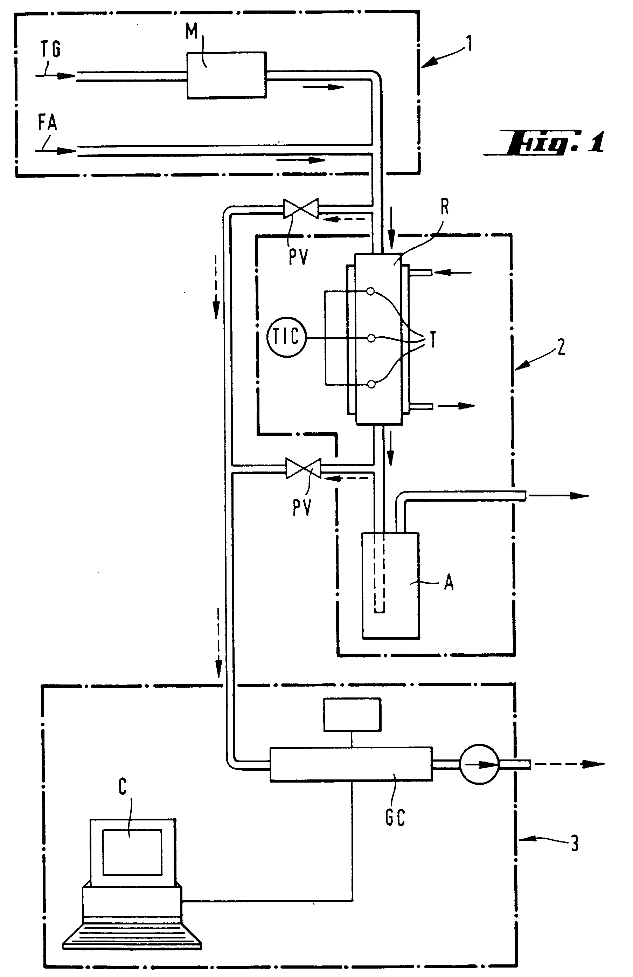

Die Apparatur zur Herstellung von Trioxan gemäß der Erfindung bestand aus drei Teilen (s. Fig. 1):

1. Dosierung
2. Bildungsreaktor
3. Analytik

Zur Untersuchung wird Formaldehyd (FA) (= Eduktstrom) in die Apparatur eingeschleust und - wenn gewünscht - mit einem Trägergas (TG) vermischt. Als Trägergas können die Edelgase Helium, Argon, Krypton oder Xenon dienen, bevorzugt ist jedoch Stickstoff.

Der Bildungsreaktor (R) bestand - für die vorliegenden Versuche - aus einem Edelstahlrohrreaktor von 150 mm Länge und einem Durchmesser von 30 mm. Die Wärmezu- bzw. -abfuhr geschah durch einen Thermostaten (TIC), als Wärmeüberträger wurde Silikonöl benutzt. Die Benutzung von Wärmeträgern wie Mineralölen ist ebenfalls möglich. An drei verschiedenen Stellen (T) des Reaktors wurde die Temperatur im Reaktor radial gemessen. Diese Temperaturen wurden während der Versuche aufgezeichnet und gaben Auskunft über den stabilen Betriebszustand des Reaktors während der Versuchsdauer. Der aus dem Reaktor austretende Ausgangsstrom mit den entstandenen Reaktionsprodukten wurde in einer Absorptionsvorrichtung (A) in Wasser aufgefangen. Das gebildete Trioxan kann daraus in bekannter Weise durch Extraktion gewonnen werden.

Die quantitativen Untersuchungen zur Bildung von Trioxan in der Gasphase wurden mit einer Online-Analytik durchgeführt. Dabei wurden an zwei verschiedenen Stellen der Apparatur während des Betriebs Proben gezogen und in einem Gaschromatographen (GC) analysiert.

Die Anordnung der Apparatur für das Verfahren gemäß der Erfindung sowie die Dimensionen können natürlich den jeweiligen Bedingungen angepaßt werden.

Versuchsbeschreibung:

Einzelne Parameter, die variiert wurden, werden bei den einzelnen Beispielen erläutert.

Bei den Versuchen zur Trimerisierung wurde wasserfreier Formaldehyd eingesetzt. Mit Hilfe eines Massendurchflußreglers (M) kann ein Trägergas (TG), vorzugsweise Stickstoff, dem Eduktstrom (FA) zugefügt werden. Es kann jedoch auch ohne Trägergas gearbeitet werden. Durch Einsatz eines Trägergases kann die Strömungsgeschwindigkeit des Eduktstromes variiert, z. B. erhöht, werden und damit die Verweilzeit im Reaktor (R) beeinflußt, z. B. erniedrigt, werden. Der Katalysator wird beispielsweise in Form von Pellets in den Reaktor geschüttet, wobei - wie in den Beispielen angegeben - verschiedene Mengen eingesetzt wurden. Vor und nach dem Reaktor (R) wurde die Gaszusammensetzung Online mit einem Gaschromatographen bestimmt. Aus den Zusammensetzungen der Gase wurde der Umsatz berechnet. Die Temperatur wurde über einen Thermostaten (TIC) geregelt und an drei verschiedenen Punkten (T) entlang der Katalysatorschüttung gemessen.

Die Probenahme zur Untersuchung der Eingangs- und Ausgangsströme wurde automatisch vorgenommen und erfolgte in regelmäßigen Abständen, z. B. alle sieben Minuten, wobei die Absperrvorrichtungen (PC) (pneumatische Kugelhähne) mit einem Gaschromatographen (GC) verbunden sind, der über einen Computer (C) gesteuert wird.

Ausbeute und Umsatz in dem Verfahren gemäß der Erfindung kann nach verschiedenen Gleichungen bestimmt werden. So wird z. B. der Molenbruch $X_{Form}$ auf die Gaszusammensetzung am Synthesereaktoreingang bezogen. Er wird nach Gleichung (1) berechnet.

$$X_{Form} = n^{\circ}_{Form}/n_{ges} = n^{\circ}_{Form}/(n^{\circ}_{Form}+n_{N2}) \tag{1}$$

| $n^{\circ}_{Form}$: | Formaldehydeingangsstrom | [mol/h] |
|---|---|---|
| $n_{ges}$ : | Gesamtstrom | [mol/h] |
| $n_{N2}$ : | Stickstoffstrom | [mol/h] |

Der experimentell ermittelte Umsatz $U_{exp}$ wird nach Gleichung (2) berechnet.

$$U_{exp} = (n^{\circ}_{Form}-n_{Form})/n^{\circ}_{Form} * 100 \tag{2}$$

| $n^{\circ}_{Form}$: | Formaldehydeingangsstrom | [mol/h] |
|---|---|---|
| $n_{Form}$ : | Formaldehydausgangsstrom | [mol/h] |

Der relative Umsatz $U_{rel}$ (Gleichung (3) ist das Verhältnis von experimentell ermitteltem Umsatz $U_{exp}$ und dem Gleichgewichtsumsatz $U_{glge}$, bestimmt aus der Gleichgewichtskonstanten nach Busfield und Merigold, ("The Gas-Phase Equilibrium between Trioxane and Formaldehyde", J. Chem. Soc (A), 1969 S. 2975).

$$U_{rel} = U_{exp}/U_{glge} *100 \tag{3}$$

| $U_{exp}$ : | experimenteller Umsatz | [%] |
|---|---|---|
| $U_{glge}$ : | Gleichgewichtsumsatz | [%] |

Die Werte der Tabellen in den Beispielen sind nach diesen Gleichungen erhalten worden.

Beispiele

1) 82 g des Katalysators Vanadylhydrogenphosphat-Hemihydrat in Form von zylindrischen Pellets (Durchmesser 5 mm, Höhe 5 mm) wurden im Reaktor (R) eingesetzt. Die Temperatur des Reaktors betrug 80°C. Folgende Umsätze konnten bei einer Selektivität von 1 erreicht werden, d.h. es wurden keine Nebenprodukte erhalten. Der Stickstoffstrom betrug 1,8 mol/h.

Tab 1.

| Umsätze bei verschiedenen Eingangsmolenbrüchen von Formaldehyd und einer Temperatur von 80°C. | | | | | | |
|---|---|---|---|---|---|---|
| Temp. [°C] | Verweilzeit [s] | $n^\circ_{Form}$ [mol/h] | Molenbruch | $U_{exp}$ [%] | $U_{rel}$ [%] | RZA [kg/m$^3$h] |
| 80 | 5,82 | 0,43 | 0,193 | 22,1 | 62,8 | 35,3 |
| 80 | 5,93 | 0,39 | 0,178 | 18,7 | 56,6 | 27,1 |
| 80 | 5,97 | 0,38 | 0,173 | 15,8 | 49,0 | 22,2 |
| 80 | 6,0 | 0,37 | 0,169 | 15,4 | 48,7 | 21,0 |

2) Beispiel 1 wurde wiederholt, wobei die Temperatur des Reaktors 90°C betrug. Folgende Umsätze konnten bei einer Selektivität von 1 erreicht werden. Der Stickstoffstrom betrug 1,8 mol/h.

Tab 2.

| Umsätze bei verschiedenen Eingangsmolenbrüchen von Formaldehyd und einer Temperatur von 90°C. | | | | | | |
|---|---|---|---|---|---|---|
| Temp. [°C] | Verweilzeit [s] | $n^\circ_{Form}$ [mol/h] | Molenbruch | $U_{exp}$ [%] | $U_{rel}$ [%] | RZA [kg/m$^3$h] |
| 90 | 5,40 | 0,52 | 0,225 | 18,0 | 81,3 | 36,3 |
| 90 | 5,85 | 0,34 | 0,160 | 16,2 | 100 | 21,5 |
| 90 | 5,90 | 0,33 | 0,153 | 15,7 | 100 | 19,7 |
| 90 | 6,11 | 0,25 | 0,123 | 13,4 | 100 | 13,0 |

Es zeigt sich, daß bei Temperaturerhöhung der Gleichgewichtsumsatz erreicht werden kann. Die Selektivität bleibt bei Temperaturerhöhung erhalten.

3) 64 g des Katalysators Vanadylhydrogenphosphat-Hemihydrat in Form von zylindrischen Pellets (Durchmesser 5 mm, Höhe 5 mm) wurden im Reaktor (R) eingesetzt. Die Temperatur des Reaktors betrug 110 °C. Folgende Umsätze konnten bei einer Selektivität von 1 erreicht werden. Der Stickstoffstrom betrug 0,1 - 0,2 mol/h. Der Gesamtdruck betrug 1700 mbar und der Eingangspartialdruck von Formaldehyd wurde bei 1100 mbar konstant gehalten.

Tab 3.

| Umsätze bei verschiedenen Eingangsmolenbrüchen von Formaldehyd und einer Temperatur von 110°C. | | | | | | |
|---|---|---|---|---|---|---|
| Temp. [°C] | Verweilzeit [s] | $n^\circ_{Form}$ [mol/h] | Molenbruch | $U_{exp}$ [%] | $U_{rel}$ [%] | RZA [kg/m$^3$h] |
| 110 | 25,4 | 0,34 | 0,717 | 23,5 | 71,3 | 31,9 |
| 110 | 34,8 | 0,25 | 0,711 | 23,7 | 73,9 | 23,3 |
| 110 | 20,8 | 0,41 | 0,695 | 17,9 | 58,0 | 29,4 |

4) Beispiel 3 wurde unter Verwendung von 21,5 g Katalysator wiederholt. Durch die Verringerung der Katalysatormasse wurde eine Verkürzung der Verweilzeit im Reaktor bewirkt. Der Gesamtdruck betrug 1350 mbar, der Eingangspartialdruck von Formaldehyd 900 mbar.

Tab 4.

| Umsätze bei verschiedenen Eingangsmolenbrüchen von Formaldehyd und einer Temperatur von 110°C. | | | | | | |
|---|---|---|---|---|---|---|
| Temp. [°C] | Verweilzeit [s] | $n^{\circ}_{Form}$ [mol/h] | Molenbruch | $U_{exp}$ [%] | $U_{rel}$ [%] | RZA [kg/m³h] |
| 110 | 6,7 | 0,47 | 0,778 | 6,2 | 25,4 | 35,1 |
| 110 | 7,9 | 0,34 | 0,673 | 7,8 | 33,1 | 32,0 |
| 110 | 8,3 | 0,29 | 0,588 | 9,9 | 39,8 | 33,8 |

5) Beispiel 3 wurde wiederholt mit dem Unterschied, daß der Gesamtdruck 1350 mbar und der Eingangspartialdruck von Formaldehyd 900 mbar betrug.

Tab 5.

| Umsätze bei verschiedenen Eingangsmolenbrüchen von Formaldehyd und einer Temperatur von 110°C. | | | | | | |
|---|---|---|---|---|---|---|
| Temp. [°C] | Verweilzeit [s] | $n^{\circ}_{Form}$ [mol/h] | Molenbruch | $U_{exp}$ [%] | $U_{rel}$ [%] | RZA [kg/m³h] |
| 110 | 14,9 | 0,61 | 0,752 | 11,6 | 45,5 | 28,4 |
| 110 | 17,9 | 0,50 | 0,751 | 14,8 | 56,5 | 29,8 |
| 110 | 24,1 | 0,37 | 0,732 | 17,8 | 66,3 | 26,1 |
| 110 | 33,0 | 0,26 | 0,725 | 20,4 | 79,5 | 21,6 |

Die Beispiele 1 bis 5 zeigen, daß der Katalysator eine hohe Selektivität für die Bildung von Trioxan besitzt. Bei einer Temperatur von 90 °C, Beispiel 2, können die höchsten relativen Umsätze erreicht werden. Jedoch sind dann nur niedrige Partialdrücke von Formaldehyd möglich. Ab einer Temperatur von 105 °C kann der Partialdruck von Formaldehyd auf über 1 bar, Beispiel 3, gesteigert werden. Es zeigt sich, daß bei längeren Verweilzeiten, z. B. 33,0 Sekunden, ein relativer Umsatz von bis 79,5 %, Beispiel 5, erreicht werden kann. Dabei verringert sich jedoch die Raumzeitausbeute. Durch eine Optimierung von Verweilzeit und dem Partialdruck von Formaldehyd, können aber Raumzeitausbeuten von über 30 kg/m³∗h erreicht werden (Beispiel 3: Verweilzeit 25,4 Sekunden, Partialdruck von Formaldehyd 1100 mbar, Raumzeitausbeute 31,9 kg/m³∗h). Bei Erniedrigung der Verweilzeit, Beispiel 4, auf 7 bis 8 Sekunden erhöht sich zwar die Raumzeitausbeute, jedoch nimmt der relative Umsatz erheblich ab.

Aktivierung von Vanadylhydrogenphosphat-Hemihydrat:

In einem mit einer elektrischen Heizmanschette auf 180°C beheizten Rohrreaktor (Durchmesser 50 mm, Länge 150 mm) wurden 150 g frisch hergestelltes Vanadylhydrogenphosphat-Hemihydrat (Korngröße 1 bis 2 mm, Schüttdichte 0,63 g/cm³) eingefüllt. Zur Aktivierung des Katalysators wurde über die Katalysatorschüttung in einem Zeitraum von 3 Tagen ein Gasstrom, bestehend aus 90 mol% Wasserdampf und 10 mol% Stickstoff, bei 1100 mbar Gesamtdruck und einem Gesamteingangsstrom von 2,57 mol/h geleitet.

Bestimmung der Aktivität von wasserdampfbehandeltem bzw. nicht aktiviertem Vanadylhydrogenphosphat-Hemihydrat:

In einen Rohrreaktor aus Edelstahl (Durchmesser 30 mm, Wandtemperatur 107,5°C) wurde eine Schüttung aus 20 cm³ (entsprechend 12,5 g frischem, beziehungsweise durch Wasserdampfbehandlung aktiviertem) Vanadylhydrogenphosphat-Hemihydrat (Korngröße 1 bis 2 mm) eingebracht und darüber ein Gasstrom, bestehend aus 0,2 mol/h Stickstoff und 0,6 mol/h wasserfreiem Formaldehyd, geleitet. Der Gesamtdruck im Reaktor betrug 1460 mbar und der Eingangspartialdruck des Formaldehyds 1100 mbar. Durch gaschromatographische Analyse wurde die Zusammensetzung des erhaltenen Produktes untersucht, das aus Stickstoff, nicht umgesetztem Formaldehyd und dem gewünschten Reaktionsprodukt Trioxan bestand.

Die Ergebnisse der Katalysatoraktivierung sind im folgenden zusammengefaßt:

6) aktiviertes Vanadylhydrogenphosphat-Hemihydrat:
Versuchsbedingungen wie oben beschrieben.

| Umsatz des Formaldehyds zu Trioxan: | 12,5 % |
|---|---|
| Raumzeitausbeute an Trioxan: | 114 kg/m$^3$ * h |

7) (Vergleich): frisches, nicht aktiviertes Vanadylhydrogenphosphat-Hemihydrat:
Versuchsbedingungen wir oben beschrieben.

| Umsatz des Formaldehyds zu Trioxan: | 2,9 % |
|---|---|
| Raumzeitausbeute an Trioxan: | 26 kg/m$^3$ * h |

Wie die Ergebnisse zeigen, steigt die Aktivität des Vanadylhydrogenphosphat-Hemihydrat bei identischen Versuchsbedingungen durch die Wasserdampfbehandlung stark an.


**Patentansprüche**

1. Verfahren zur Herstellung von Trioxan aus Formaldehyd in der Gasphase in Gegenwart eines Katalysators, dadurch gekennzeichnet, daß als Katalysator Vanadylhydrogenphosphat-Hemihydrat eingesetzt wird.

2. Verfahren nach Anspruch 1, dadurch gekennzeichnet, daß der Katalysator unter Reaktions bedingungen als eine wasserärmere Form von Vanadylhydrogenphosphat-Hemihydrat vorliegt.

3. Verfahren nach Anspruch 1, dadurch gekennzeichnet, daß der Katalysator vor seinem Einsatz durch Behandlung mit Wasserdampf aktiviert wird.

4. Verfahren nach Anspruch 1 oder 3, dadurch gekennzeichnet, daß ein Katalysator eingesetzt wird, der bei 100 bis 220°C mit Wasserdampf in An- oder Abwesenheit eines Trägergases für 24 Stunden bis 14 Tage behandelt wird, und die Behandlung drucklos oder unter Anwendung von erhöhtem Druck erfolgt.

5. Verfahren nach einem der Ansprüche 1 bis 4, dadurch gekennzeichnet, daß wasserfreier Formaldehyd eingesetzt wird.

6. Verfahren nach einem der Ansprüche 1 bis 5, dadurch gekennzeichnet, daß die Reaktion bei 80 bis 150°C, vorzugsweise bei 100 bis 120°C durchgeführt wird.

7. Verfahren nach einem der Ansprüche 1 bis 6, dadurch gekennzeichnet, daß der Eingangspartialdruck des Formaldehyds 0,5 bis 5 bar, vorzugsweise 0,5 bis 2 bar beträgt.

8. Verfahren nach einem der Ansprüche 1 bis 7, dadurch gekennzeichnet, daß die Reaktion in Anwesenheit oder Abwesenheit eines Trägergases durchgeführt wird.

9. Verfahren nach Anspruch 8, dadurch gekennzeichnet, daß Stickstoff als Trägergas verwendet wird.


**Claims**

1. A process for the preparation of trioxane from formaldehyde in the gas phase in the presence of a catalyst, wherein the catalyst employed is vanadyl hydrogenphosphate hemihydrate.

2. The process as claimed in claim 1, wherein, under the reaction conditions, the catalyst exists as a relatively low-water-content form of vanadyl hydrogenphosphate hemihydrate.

3. The process as claimed in claim 1, wherein the catalyst is activated by treatment with steam prior to being employed.

4. The process as claimed in claim 1 or 3, wherein the catalyst employed is treated at from 100 to 220°C with steam in the presence or absence of a carrier gas for from 24 hours to 14 days, and the treatment is carried out without pressure or with application of increased pressure.

5. The process as claimed in any one of claims 1 to 4, wherein anhydrous formaldehyde is employed.

6. The process as claimed in any one of claims 1 to 5, wherein the reaction is carried out at from 80 to 150°C, preferably at from 100 to 120°C.

7. The process as claimed in any one of claims 1 to 6, wherein the partial pressure of the formaldehyde at the inlet is from 0.5 to 5 bar, preferably from 0.5 to 2 bar.

8. The process as claimed in any one of claims 1 to 7, wherein the reaction is carried out in the presence or absence of a carrier gas.

9. The process as claimed in claim 8, wherein the carrier gas used is nitrogen.

**Revendications**

1. Procédé pour la préparation du trioxane à partir de formaldéhyde en phase gazeuse en présence d'un catalyseur, caractérisé en ce que l'on utilise l'hémihydrate de l'hydrogénophosphate de vanadyle comme catalyseur.

2. Procédé selon la revendication 1, caractérisé en ce que le catalyseur est présent dans les conditions réactionnelles sous forme pauvre en eau d'hémihydrate de l'hydrogénophosphate de vanadyle.

3. Procédé selon la revendication 1, caractérisé en ce que le catalyseur est activé par traitement à la vapeur d'eau avant son utilisation.

4. Procédé selon la revendication 1 ou 3, caractérisé en ce que l'on utilise un catalyseur que l'on traite à une température de 100 à 220 °C à la vapeur d'eau en présence ou en l'absence d'un gaz porteur pendant une durée allant de 24 heures à 14 jours et en ce que l'on effectue le traitement sans pression ou en utilisant une pression élevée.

5. Procédé selon l'une des revendications 1 à 4, caractérisé en ce que l'on utilise du formaldéhyde anhydre.

6. Procédé selon l'une des revendications 1 à 5, caractérisé en ce que l'on met en oeuvre la réaction à une température de 80 à 150 °C, de préférence à 100 à 120 °C.

7. Procédé selon l'une des revendications 1 à 6, caractérisé en ce que la pression partielle d'entrée du formaldéhyde est de 0,5 à 5 bars, de préférence de 0,5 à 2 bars.

8. Procédé selon l'une des revendications 1 à 7, caractérisé en ce que l'on met en oeuvre la réaction en l'absence ou en présence d'un gaz porteur.

9. Procédé selon la revendication 8, caractérisé en ce que l'on utilise l'azote comme gaz porteur.

**Fig. 1**